# EUROPEAN PATENT SPECIFICATION

(11) **EP 2 641 402 B1**
(45) Date of publication and mention of the grant of the patent: **15.12.2021**
(21) Application number: 11791496.0
(22) Date of filing: 18.11.2011
(51) Int. Cl.: H04N 13/25

(54) **VISUALISATION OF IMAGES AND VIDEO FOR AUTOSTEREOSCOPIC DISPLAY**
VISUALISIERUNG VON BILDERN UND VIDEOS FÜR EINE AUTOSTEREOSKOPISCHE ANZEIGE
VISUALISATION D'IMAGES ET DE VIDÉO POUR AFFICHAGE AUTOSTÉRÉOSCOPIQUE

(30) Priority: 19.11.2010 GB 201019694
(43) Date of publication of application: 25.09.2013
(73) Proprietor: Setred Medical Technology AS, 0349 Oslo (NO)
(72) Inventor: ERICSON, Lars Thomas Kristian, S-167 33 Bromma (SE); MOLLER, Christian Nicolai, N-0874 Oslo (NO)
(74) Representative: Roberts, Gwilym Vaughan
(86) International application number: PCT/EP2011/070502
(87) International publication number: WO 2012/066138

(56) References cited:
- WO-A1-2007/119063
- WO-A1-2010/058354
- US-A1- 2006 007 246
- US-A1- 2006 007 246
- US-A1- 2009 267 958
- Daniel Ruijters: "Multi-modal image fusion during minimally invasive treatment", , 15 February 2010 (2010-02-15), XP055505627, Retrieved from the Internet: URL:https://pure.tue.nl/ws/portalfiles/por tal/2931756 [retrieved on 2018-09-07]
- Ziv Yaniv ET AL: "Image-Guided Procedures: A Review", Computer Aided Interventions and Medical Robotics, 1 April 2006 (2006-04-01), XP055505784, Retrieved from the Internet: URL:http://yanivresearch.info/writtenMater ial/CAIMR-TR-2006-3.pdf [retrieved on 2018-09-10]
- Michael Emmanuel Leventon: "A Registration, Tracking, and Visualization System for Image-Guided Surgery", , 2 May 1997 (1997-05-02), XP055038007, Retrieved from the Internet: URL:https://www.spl.harvard.edu/archive/sp l-pre2007/pages/papers/leventon/thesis/mas ter/thesis.pdf [retrieved on 2012-09-12]
- Daniel Ruijters: "Multi-modal image fusion during minimally invasive treatment", , 15 February 2010 (2010-02-15), XP055505627, Retrieved from the Internet: URL:https://pure.tue.nl/ws/portalfiles/por tal/2931756 [retrieved on 2018-09-07]
- Ziv Yaniv ET AL: "Image-Guided Procedures: A Review", Computer Aided Interventions and Medical Robotics, 1 April 2006 (2006-04-01), XP055505784, Retrieved from the Internet: URL:http://yanivresearch.info/writtenMater ial/CAIMR-TR-2006-3.pdf [retrieved on 2018-09-10]
- Michael Emmanuel Leventon: "A Registration, Tracking, and Visualization System for Image-Guided Surgery", , 2 May 1997 (1997-05-02), XP055038007, Retrieved from the Internet: URL:https://www.spl.harvard.edu/archive/sp l-pre2007/pages/papers/leventon/thesis/mas ter/thesis.pdf [retrieved on 2012-09-12]

## Description

The present invention relates to capturing and visualising images and video for an autostereoscopic display apparatus.

### Background

A well proven method for creating a 3D image is to cause a viewer to see different perspective views of a scene with each eye. One way to do this is to display two differently polarized images on a screen, and for the viewer to wear corresponding polarizing filters on each eye.

An autostereoscopic display or a three dimensional (3D) display may be implemented using an aperture or slit array in conjunction with a two dimensional (2D) display to display a 3D image. The principle of the device is that when looking at a 2D image through a slit array, the slit array separated from the screen by a distance, then the viewer sees a different part of the 2D image with each eye. If an appropriate image is rendered and displayed on the 2D display, then a different perspective image can be displayed to each eye of the viewer without necessitating them to wear filters over each eye.

Autostereoscopic displays provide additional challenges and opportunities with regards to the image acquisition and presentation. The present invention addresses some of these.

Examples of the disclosure are directed towards capturing images for an autostereoscopic display and visualising the images along with other 3D data on an autostereoscopic display. The invention disclosed herein may be implemented in the scanning slit time-multiplexed system described in PCT application PCT/IB2005/001480. There is a wide range of application areas, while medical imaging will be used as an example.

Autostereoscopic and lightfield displays that allow viewers to move freely are typically multi-perspective displays, i.e. they provide at least two but in many instances many more perspectives. Methods for capturing multiple and even continuous perspectives directly exist in the form of so called light field sensors. These may for example use a lenticular array between the scene being recorded and the imaging sensor. While these methods are not covered in detail in this document they may be used in combination with methods presented herein.

Other methods for capturing and presenting multiple perspectives include calculating, for example through interpolation, perspectives from cameras that capture one or several perspective views, but fewer views than the display can show. Many techniques for this exist mainly based on using a depth or disparity map along with original images. Depth map can be obtained through processing the images themselves or by additional sensors that obtain the depth map directly from the scene being recorded.

These document are also referred to: US2009267958 WO2007119063 WO2010058354 and the articles by Daniel Ruijters, "Multi-modal image fusion during minimally invasive treatment", (20100215), URL: https://pure.tue.nl/ws/portalfiles/portal/2931756, (20180907), Ziv Yaniv ET AL, "Image-Guided Procedures: A Review", Computer Aided Interventions and Medical Robotics, (20060401), URL: http://yanivresearch.info/writtenMaterial/CAIMR-TR-2006-3.pdf, (20180910), and Michael Emmanuel Leventon, "A Registration, Tracking, and Visualization System for Image-Guided Surgery", (19970502), URL: https://www.spl.harvard.edu/archive/spl-pre2007/pages/papers/leventon/thesis/master/thesis.pdf, (20120912)

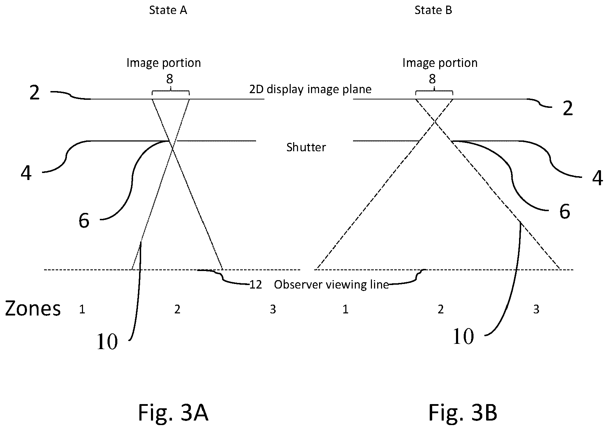

The invention is set out in the appended claims. In particular, the invention as defined in the independent claims correspond to figures 3A and 3B and the corresponding passages of the description.

### Description

The display properties for a viewing position can be changed during operation of the display.

The different representations of the three dimensional information may be the result of different recording methods for the same three dimensional information (e.g. pressure and temperature measurements of a three dimensional space; different statistical samples of the same population etc) and/or different image capture methods (such as but not limited to: computed tomography; magnetic resonance imaging; ultrasound; optical camera; depth camera; laser surface scanning; positron emission tomography; and x-ray).

Some (for instance at least two) of the representations may be the result of different processing of the same original representation (e.g. different transfer functions for a CT or different image filtering of a camera image). The representations may provide perspectives that are matched to the viewer position where the representation is shown. The matching may give the effect that when the viewer moves and experiences parallax the scene appears to be stationary while the representation of the scene changes.

The three dimensional information typically relates to a real three dimensional object or scene.

In a further embodiment, an autostereoscopic display apparatus or a light field display apparatus is arranged to provide a first set of images to a first viewing position and a second set of images to a second viewing position, the second set of images being derived from the first set of images. The images captured directly from a camera may have higher image quality or less latency than images derived from those directly captured images. In some situations it may be advantageous to present both types of images to the viewer. One way to achieve this is to provide at least one view zone where at least one directly captured image is shown and to show derived images for other view positions. The images may be derived by interpolation or using other known techniques.

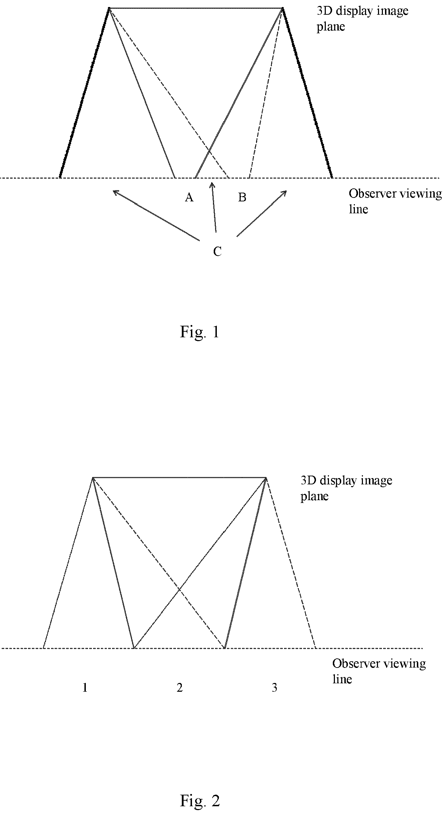

Figure 1 shows an example where two cameras have been used to provide two directly captured images. In zone A of the view line images from camera A are shown, in zone B images from camera B are shown, and in the other zones C derived images are shown. The zones A and B may be positioned such that the position of the centre of each zone have the same separation in relation to the distance to the conversion plane and/or the focus plane of the 3D display as the cameras to the plane of focus in the scene being captured. Derived images may be provided with the same or different angular perspective separation as the directly captured images. In many instances the 3D quality is improved by smaller perspective separation and derived images may be provided with the minimum perspective separation that the 3D display can reproduce.

In some applications it may be beneficial to track the position of the viewers head as to shift the zones with the directly captures image to be located at the viewers head or eye positions. One may also provide a head rest located such that it ensures that one or several viewer's eyes are seeing directly captured images. The head rest may be tracked such that the zones can be shifted when the head rest is moved.

In order to give minimum latency for directly captured images these could be given a separate image channel from the camera to the display imaging device. The directly captured images may then also be sent in a separate channel to the image processing device that calculates the derived images and then via a channel to the display imaging device.

Another method is to have a user switch to switch between a first mode where only directly captured images in zones corresponding to camera positions are shown and a second mode where all zones show only derived images.

In some instances there will be more than one type of image data that represent different aspects of the same scene. This can be directly captured images based on different types of cameras, e.g. a standard camera and an IR camera. It may also be data from other imaging sources such as CT, MRI and ultrasound. The 3D display and the capturing system may provide several ways of effectively representing the different types of data. The methods described can be used stand alone or as a combination of the different methods.

Figure 2 shows an intuitive switch between viewing different data and different filtering of data by showing them in different view zones. An autostereoscopic display is effectively a directional display that can show different images to different directions in one or more viewer zones. The example shows zone 1 where direct or derived camera perspectives 1-50 are shown, zone 2 shows equivalent perspectives 51-100 based on computed tomography data of the same scene, and zone 3 IR camera perspectives 101-150. The zones do not need to be distinct as shown in this example. One could instead do a gradual transition from one type of data or filtering to another. For example, two transfer functions for a CT dataset could be interpolated between perspective 1 and 150 such that each perspective presented to the user has a unique transfer function. Another example would be to show directly captured camera images in zones A and B in Figure 1, and corresponding perspectives of rendered CT images in zones C. One may also show no information in zones C in order to avoid inverted images in repeated zones that some autostereoscopic and lightfield displays have.

The optimal display properties may be different for different types of data. For example, camera images may require higher colour bit depth than images rendered from a CT dataset. The display may thus be set to provide different properties for different viewing zones, view direction etc. One way to achieve a higher colour bit depth for a central viewing zone, e.g. zone 2 in Figure 2, than for other viewing zones, e.g. zones 1 and 3 in Figure 2 will be explained with reference to a scanning slit time multiplexed system using a time multiplexed 2D display to provide levels of colour and greyscale. One may operate the shutter such that the viewing cone when showing the least significant bit on the 2D display is equivalent to zone 1, while the viewing cone for all other bits is the full width of zones 1, 2 and 3 together. This is illustrated in Figures 3A and 3B. In state A as shown in Figure 3A only the least significant bit is shown on the 2D display 2 and the shutter 4 has a narrow slit 6 open such that the image portion 8 on the 2D display 2 will only be seen in zone 2 on the observer viewing line 12. In state B, as shown in Figure 3B, all other bits are shown on the 2D display 2 and the shutter has 4 a wider slit 6 open such that the image portion 8 is seen in all of zones 1, 2 and 3. The viewing cone 10 may be defined as the range of viewing angles where the viewer sees a part of the 2D display 2 through a given open slit 6 where this part of the 2D display 2 belongs to the image on the 2D display 2 associated with the same open slit 6, illustrated as the image portion 8 in Figure 3.

A number of methods could be used to ensure that camera images and data from other imaging sources or other cameras are accurately aligned. One way is to have known and pre-calibrated information on the relative position and angles between the image capture devices, in addition to information on how each image capture device's position relates to the position of the image data that it captures. For example an MR scanner may provide information on where a captured voxel is positioned relative to the scanner itself, and a microscope that is attached rigidly to the MR may provide information on the position of its focus point. By knowing the relative position of the MR scanner and the microscope, the relative position of a voxel and the focus point of the microscope may be determined. A more flexible approach involves continuously tracking the relative position of the image capture devices. In medical procedures it is common to use navigation systems to track both the patient and the camera and to co-register these with other imaging data such as CT, MRI or ultrasound. Tracking can be done is several ways with common methods being through depth cameras, comprising two or more normal or IR cameras or through using electromagnetic tracking. Another method is to use cameras using time of flight technology to obtain the depth for each pixel in an image. Light field cameras that can capture information on the properties of incoming light from different directions in a given point may also be used to obtain depth information about a scene and objects within it.

When capturing a static scene, a nearly static scene or a scene where the relevant objects are static one may collect more extensive information about the scene by combining information from two or more instances in time. This is for example used when creating a 3D volume using a 2D ultrasound probe. The 2D ultrasound probe captures a 2D plane of information from an object, e.g. a patient. The probe is then moved and the movement is tracked by one of the tracking methods mentioned above. This captures information on the volume swept when the 2D plane moves through the relevant parts of the object. Similarly a camera providing a depth image of a scene, i.e. information on both colour and depth for one or several points in the scene, may be tracked such that a composite image or 3D model can be created both by moving the depth camera sideways and by capturing the depth images from different angles of the scene or object. One may also do an overlay such that one type of 3D data such as CT, MRI images can be shown at the same time for one or several view directions as another type of data such as the camera data that may come from a microscope or camera. One way to achieve this is to use a navigation system that tracks the camera and the scene being captured, such as the patient. A standard process is then used to co-register the patient position with the CT, MRI or other imaging data of the patient. By using the depth map derived from any of the methods mentioned above it is possible to know the position of a pixel in the camera image in the co-ordinate system of the tracing system and hence to render it in the correct position and show it simultaneously as for example the CT data.

One problem facing surgeons using navigation systems is that the brain shifts during surgery compared to its position when the image data from CT, MRI, ultrasound etc were taken. The example below will make reference to a CT and a camera, but the same method may be applied to any combination of image capture devices. One way to overcome this problem is to transform the image data based on direct observations during surgery. A simple transformation would be to shift, rotate or scale all points in the CT volume. This may not be correct since the shift depends on the type of substance or tissue as well as the position in the brain. By modelling those parameters it is possible to more accurately calculate a transformation on how structures and substances in the brain will shift. Accuracy may also be improved by matching internal anatomical landmarks in the brain as seen directly or through the use of a camera or microscope with the same landmarks in the CT. This may be achieved by selecting a pixel on a landmark in the camera image, since the tracking described above can give the position of a pixel in the tracked co-ordinate system, and matching the selected point with the same point on the landmark based on the CT image. Another method may include using automatic or semi-automatic image co-registration methods. There are a number of available algorithms available for such co-registration based on the image information in two or more 3D volumes, and such methods are for example used for co-registration of CT and MR images of the same patient. The methods may both be based on rigid body assumptions and provide only translation, rotation and scaling of a 3D volume or scene. They may also allow for non-rigid transformations where additional relative transformations within the same 3D volume or scene are allowed. The co-registration may be followed by a visual confirmation step by the viewer and may include the possibility to make manual adjustments. One may also use other methods such as using a pointing tool that is tracked and point it to the landmark. Combining the methods described in this paragraph may improve accuracy further.

In a further embodiment, at least two different image capturing methods may be used to capture a three dimensional object or scene. The captured information is visualised on an autostereoscopic or lightfield display. Data points from each image capturing method are then used to verify and adjust the correct relative positions and transformations of the images captured using the different capturing methods.

The relative position and angular position of at least two of the image capturing devices or at least one image capturing device and the real object or scene may be tracked. This may have particular application where the real object is a human body and the data points are selected based on anatomical landmarks on the patient. The adjusting and verifying the correct relative positions and transformations may be an automatic or semi-automatic co-registration method based on the image information.

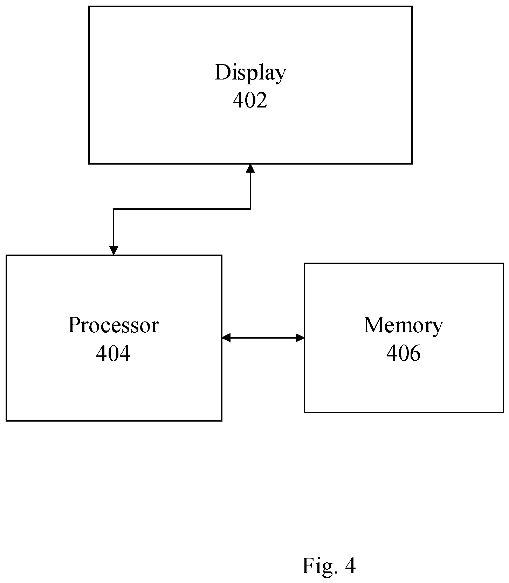

Figure 4 is a block diagram of an autostereoscopic display apparatus or a light field display apparatus. The autostereoscopic display apparatus or a light field display apparatus comprises: a display 402; a processor 404; and a memory 406 having stored therein one or more routines executable by the processor, the one or more routines being adapted to operate according to one or more of the methods described herein.

A computer-readable medium may also be provided having computer-executable instructions adapted to cause an autostereoscopic display apparatus or a light field display apparatus comprising a scanning slit time multiplexed system using a time multiplexed 2D display to perform one or more of the methods described herein.

## Claims

1. A method of operating an autostereoscopic display apparatus or a light field display apparatus comprising a scanning slit time multiplexed system using a time multiplexed 2D display,
wherein the method comprises:
showing at least two different representations of three dimensional information to at least two different viewer positions; and
providing a different display property to each of the different viewer positions,
wherein the display property is colour bit depth, and
wherein providing a different colour bit depth to each of the different viewer positions comprises:
providing a first colour bit depth to a first viewer position and a second colour bit depth to a second viewer position, the first colour bit depth being defined by opening a slit of the scanning slit time multiplexed system, the slit having a first width, and presenting on the 2D display the least significant bit of a first representation of the three dimensional information, and the second colour bit depth being defined by opening a slit of the scanning slit time multiplexed system, the slit having a second width, and presenting on the 2D display all other bits of a second representation of the three dimensional information,
wherein the first width is narrower than the second width.

2. The method according to claim 1, wherein the display properties for a viewing position can be changed during operation of the display.

3. The method according to claim 1, wherein the at least two different representations of the three dimensional information are the result of different recording methods for the same three dimensional information.

4. The method according to claim 1, wherein the three dimensional information relates to a three dimensional object or scene and the different representations are different image capture methods.

5. The method according to claim 4, wherein the image capture methods include at least one of the following:
computed tomography;
magnetic resonance imaging;
ultrasound;
optical camera;
positron emission tomography; and
x-ray.

6. The method according to any preceding claim where at least two different representations are the result of different processing of the same original representation.

7. The method according to any preceding claims where the representations provide perspectives that are matched to the viewer position where the representation is shown.

8. An autostereoscopic display apparatus or a light field display apparatus comprising a scanning slit time multiplexed system using a time multiplexed 2D display, the apparatus further comprising:
a processor; and
a memory having stored therein one or more routines executable by the processor, the one or more routines being adapted to operate according to the method of any of claims 1 to 7.

9. A computer-readable medium having computer-executable instructions adapted to cause an autostereoscopic display apparatus or a light field display apparatus comprising a scanning slit time multiplexed system using a time multiplexed 2D display to perform the method of any of claims 1 to 7.

10. A data carrier carrying thereon or therein data indicative of instructions executable by processing means of an autostereoscopic display apparatus or a light field display apparatus comprising a scanning slit time multiplexed system using a time multiplexed 2D display to cause those means to carry out a method according to any of claims 1 to 7.

## Patentansprüche

1. Verfahren zum Betreiben einer autostereoskopischen Anzeigevorrichtung oder einer Lichtfeld-Anzeigevorrichtung, die ein Zeitmultiplex-System mit Abtastspalt unter Verwendung einer Zeitmultiplex-2D-Anzeige umfasst,
wobei das Verfahren Folgendes umfasst:
Zeigen von mindestens zwei unterschiedlichen Darstellungen von dreidimensionalen Informationen an mindestens zwei unterschiedlichen Betrachterpositionen und
Bereitstellen einer unterschiedlichen Anzeigeeigenschaft an jeder der unterschiedlichen Betrachterpositionen, wobei die Anzeigeeigenschaft eine Farbtiefe ist,
wobei das Bereitstellen einer unterschiedlichen Farbtiefe an jeder der unterschiedlichen Betrachterpositionen Folgendes umfasst:
Bereitstellen einer ersten Farbtiefe an einer ersten Betrachterposition und einer zweiten Farbtiefe an einer zweiten Betrachterposition, wobei die erste Farbtiefe durch Öffnen eines Spalts des Zeitmultiplex-Systems mit Abtastspalt definiert ist, wobei der Spalt eine erste Breite aufweist, und auf der 2D-Anzeige das niedrigstwertige Bit einer ersten Darstellung der dreidimensionalen Informationen präsentiert und die zweite Farbtiefe durch Öffnen eines Spalts des Zeitmultiplex-Systems mit Abtastspalt definiert ist, wobei der Spalt eine zweite Breite aufweist, und auf der 2D-Anzeige alle anderen Bits einer zweiten Darstellung der dreidimensionalen Informationen präsentiert,
wobei die erste Breite schmaler als die zweite Breite ist.

2. Verfahren nach Anspruch 1, wobei die Anzeigeeigenschaften für eine Betrachtungsposition während des Betriebs der Anzeige geändert werden können.

3. Verfahren nach Anspruch 1, wobei die mindestens zwei unterschiedlichen Darstellungen der dreidimensionalen Informationen das Ergebnis von unterschiedlichen Aufzeichnungsverfahren für dieselben dreidimensionalen Informationen sind.

4. Verfahren nach Anspruch 1, wobei sich die dreidimensionalen Informationen auf ein dreidimensionales Objekt oder eine Szene beziehen und die unterschiedlichen Darstellungen unterschiedliche Bildaufnahmeverfahren sind.

5. Verfahren nach Anspruch 4, wobei die Bildaufnahmeverfahren mindestens eines des Folgenden umfassen:
Computertomographie;
Kernspintomographie;
Ultraschall;
optische Kamera;
Positronen-Emissions-Tomographie und
Röntgenstrahlen.

6. Verfahren nach einem der vorhergehenden Ansprüche, wobei mindestens zwei unterschiedliche Darstellungen das Ergebnis von unterschiedlicher Verarbeitung derselben originalen Darstellung sind.

7. Verfahren nach einem der vorhergehenden Ansprüche, wobei die Darstellungen Perspektiven bereitstellen, die mit der Betrachterposition, an der die Darstellung gezeigt wird, in Übereinstimmung gebracht werden.

8. Autostereoskopische Anzeigevorrichtung oder Lichtfeld-Anzeigevorrichtung, die ein Zeitmultiplex-System mit Abtastspalt unter Verwendung einer Zeitmultiplex-2D-Anzeige umfasst, wobei die Vorrichtung ferner Folgendes umfasst:
einen Prozessor und
einen Speicher, der eine oder mehrere Routinen gespeichert hat, die durch den Prozessor ausgeführt werden können, wobei die eine oder die mehreren Routinen dafür ausgelegt sind, gemäß dem Verfahren nach einem der Ansprüche 1 bis 7 zu arbeiten.

9. Computerlesbares Medium, das computerausführbare Anweisungen aufweist, die dafür ausgelegt sind, zu bewirken, dass eine autostereoskopische Anzeigevorrichtung oder eine Lichtfeld-Anzeigevorrichtung, die ein Zeitmultiplex-System mit Abtastspalt unter Verwendung einer Zeitmultiplex-2D-Anzeige umfasst, das Verfahren nach einem der Ansprüche 1 bis 7 durchführt.

10. Datenträger, der Daten trägt, die Anweisungen angeben, die durch Verarbeitungsmittel einer autostereoskopischen Anzeigevorrichtung oder einer Lichtfeld-Anzeigevorrichtung, die ein Zeitmultiplex-System mit Abtastspalt unter Verwendung einer Zeitmultiplex-2D-Anzeige umfasst, ausgeführt werden können, um zu bewirken, dass diese Mittel ein Verfahren nach einem der Ansprüche 1 bis 7 ausführen.

## Revendications

1. Procédé de fonctionnement d'un appareil d'affichage autostéréoscopique ou d'un appareil d'affichage de champ lumineux comprenant un système à multiplexage temporel de fente de balayage utilisant un affichage 2D à multiplexage temporel,
où le procédé comprend :
de montrer au moins deux représentations différentes d'informations tridimensionnelles à au moins deux positions différentes de l'observateur ; et
de fournir une propriété d'affichage différente à chacune des différentes positions de l'observateur,
où la propriété d'affichage est la profondeur de bit de couleur, et
où la fourniture d'une profondeur de bit de couleur différente à chacune des différentes positions de l'observateur comprend :
de fournir une première profondeur de bit de couleur à une première position de l'observateur et une seconde profondeur de bit de couleur à une seconde position de l'observateur, la première profondeur de bit de couleur étant définie par l'ouverture d'une fente du système à multiplexage temporel de fente de balayage, la fente ayant une première largeur, et de présenter sur l'affichage 2D le bit le moins significatif d'une première représentation des informations tridimensionnelles, et la seconde profondeur de bit de couleur étant définie par l'ouverture d'une fente du système à multiplexage temporel de fente de balayage, la fente ayant une seconde largeur, et de présenter sur l'affichage 2D tous les autres bits d'une seconde représentation des informations tridimensionnelles,
où la première largeur est plus étroite que la seconde largeur.

2. Procédé selon la revendication 1, dans lequel les propriétés d'affichage pour une position de visualisation peuvent être modifiées pendant le fonctionnement de l'affichage.

3. Procédé selon la revendication 1, dans lequel les au moins deux représentations différentes des informations tridimensionnelles sont le résultat de différents procédés d'enregistrement pour les mêmes informations tridimensionnelles.

4. Procédé selon la revendication 1, dans lequel les informations tridimensionnelles concernent un objet tridimensionnel ou une scène tridimensionnelle, et les différentes représentations sont des procédés de capture d'image différents.

5. Procédé selon la revendication 4, dans lequel les procédés de capture d'image comprennent au moins l'un des procédés suivants :
la tomographie assistée par ordinateur ;
l'imagerie par résonance magnétique ;
l'échographie ;
la caméra optique ;
la tomographie par émission de positrons ; et
la radiographie.

6. Procédé selon l'une quelconque des revendications précédentes, dans lequel au moins deux représentations différentes sont le résultat de traitements différents de la même représentation originale.

7. Procédé selon l'une quelconque des revendications précédentes, dans lequel les représentations fournissent des perspectives qui sont adaptées à la position de l'observateur où la représentation est montrée.

8. Appareil d'affichage autostéréoscopique ou appareil d'affichage de champ lumineux comprenant un système à multiplexage temporel de fente de balayage utilisant un affichage 2D à multiplexage temporel, l'appareil comprenant en outre :
un processeur ; et
une mémoire dans laquelle sont stockées une ou plusieurs routines exécutables par le processeur, les une ou plusieurs routines étant adaptées pour fonctionner selon le procédé de l'une quelconque des revendications 1 à 7.

9. Support lisible par ordinateur comprenant des instructions exécutables par ordinateur adaptées pour amener un appareil d'affichage autostéréoscopique ou un appareil d'affichage de champ lumineux comprenant un système à multiplexage temporel de fente de balayage utilisant un affichage 2D à multiplexage temporel pour exécuter le procédé de l'une quelconque des revendications 1 à 7.

10. Support de données supportant ou intégrant des données indicatives d'instructions exécutables par des moyens de traitement d'un appareil d'affichage autostéréoscopique ou d'un appareil d'affichage de champ lumineux comprenant un système à multiplexage temporel de fente de balayage utilisant un affichage 2D à multiplexage temporel pour amener ces moyens à exécuter un procédé selon l'une quelconque des revendications 1 à 7.
